(19) Europäisches Patentamt
European Patent Office
Office européen des brevets

(11) Veröffentlichungsnummer: 0 272 555
A2

(12) # EUROPÄISCHE PATENTANMELDUNG

(21) Anmeldenummer: 87118374.5

(22) Anmeldetag: 11.12.87

(51) Int. Cl.4: C07D 261/02 , C07D 413/06 , A01N 43/80 , C07C 83/00 , C07C 149/42

(30) Priorität: 22.12.86 DE 3643942

(43) Veröffentlichungstag der Anmeldung: 29.06.88 Patentblatt 88/26

(84) Benannte Vertragsstaaten: AT BE CH DE ES FR GB IT LI NL

(71) Anmelder: BAYER AG
D-5090 Leverkusen(DE)

(72) Erfinder: Weissmüller, Joachim, Dr.
Carl-Langhans-Strasse 53
D-4019 Monheim(DE)
Erfinder: Berg, Dieter, Dr.
Gellertweg 27
D-5600 Wuppertal 1(DE)
Erfinder: Hänssler, Gerd, Dr.
Am Arenzberg 58a
D-5090 Leverkusen 3(DE)
Erfinder: Reinecke, Paul, Dr.
Steinstrasse 8
D-5090 Leverkusen 3(DE)

(54) **Aminomethylisoxazolidine.**

(57) Neue Aminomethylisoxazolidine der Formel (I)

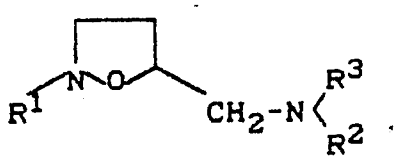

(I)

in welcher
$R^1$, $R^2$ und $R^3$ die in der Beschreibung gegebenen Bedeutungen haben, deren Säureadditionssalze, geometrische und optische Isomere sowie deren Isomerengemische und ihre Verwendung in Schädlingsbekämpfungsmitteln.

Die neuen Aminomethylisoxazolidine der Formel (I) können nach bekannten Verfahren hergestellt werden, z.B. dadurch, daß man geeignete Hydroxylamino-Derivate mit geeigneten Allylaminen in Gegenwart von Formaldehyd umsetzt oder geeignete Isoxazolidine mit geeigneten Aminen umsetzt oder geeignete Aminomethylisoxazolidine alkyliert.

EP 0 272 555 A2

## Aminomethylisoxazolidine

Die Erfindung betrifft neue Aminomethylisoxazolidine, mehrere Verfahren zu ihrer Herstellung sowie ihre Verwendung als Schädlingsbekämpfungsmittel.

Es ist bereits bekannt, daß bestimmte Isoxazolidine, wie beispielsweise das 2-Methyl-5-(2-pyridyl)-isoxazolidin oder das 5-(3,4-Dichlorbenzyl)-5-ethoxycarbonyl-2-isopropyl-isoxazolidin oder das 5-Benzyl-5-ethoxycarbonyl-2-t-butyl-isoxazolidin fungizide Eigenschaften besitzen (vgl. DE-OS 34 18 395).

Die Wirksamkeit dieser vorbekannten Verbindungen ist jedoch insbesondere bei niedrigen Aufwandmengen und Konzentrationen nicht in allen Anwendungsgebieten völlig zufriedenstellend.

Es wurden neue Aminomethylisoxazolidine der allgemeinen Formel (I),

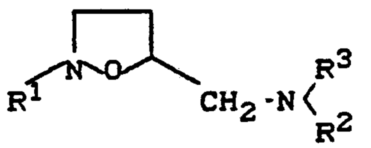

(I)

in welcher

$R^1$ für gegebenenfalls substituiertes Alkyl, für gegebenenfalls substituiertes Cycloalkyl, für gegebenenfalls substituiertes Tetrahydronaphthyl oder für gegebenenfalls substituiertes Decahydronaphthyl steht und

$R^2$ und $R^3$ unabhängig voneinander jeweils für Wasserstoff, oder für jeweils gegebenenfalls substituiertes Alkyl, Alkenyl, Cycloalkyl oder Aryl stehen oder gemeinsam mit dem Stickstoffatom, an welches sie gebunden sind, für einen gegebenenfalls substituierten, gesättigten Heterocyclus stehen, der gegebenenfalls weitere Heteroatome enthalten kann,

sowie deren Säureadditionssalze, gefunden.

Die Verbindungen der Formel (I) können gegebenenfalls als geometrische und/oder optische Isomere oder Isomerengemische unterschiedlicher Zusammensetzung anfallen. Sowohl die reinen Isomeren als auch die Isomerengemische werden erfindungsgemäß beansprucht und im folgenden unter der Formel (I) verstanden.

Weiterhin wurde gefunden, daß man die neuen Aminomethylisoxazolidine der allgemeinen Formel (I),

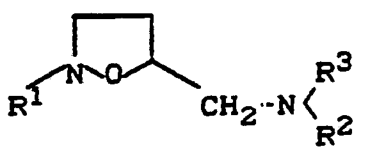

(I)

in welcher

$R^1$ für gegebenenfalls substituiertes Alkyl, für gegebenenfalls substituiertes Cycloalkyl, für gegebenenfalls substituiertes Tetrahydronaphthyl oder für gegebenenfalls substituiertes Decahydronaphthyl steht und

$R^2$ und $R^3$ unabhängig voneinander jeweils für Wasserstoff, für jeweils gegebenenfalls substituiertes Alkyl, Alkenyl, Cycloalkyl oder Aryl stehen oder gemeinsam mit dem Stickstoffatom, an welches sie gebunden sind, für einen gegebenenfalls substituierten, gesättigten Heterocyclus stehen, der gegebenenfalls weitere Heteroatome enthalten kann,

sowie deren Säureadditionssalze erhält, wenn man

(a) Hydroxylamin-Derivate der Formel (II),

$R^1$-NH-OH (II)

in welcher

$R^1$ die oben angegebene Bedeutung hat,

mit Allylaminen der Formel (III),

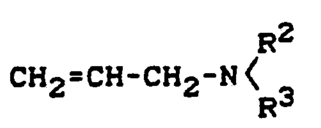

(III)

in welcher

$R^2$ und $R^3$ die oben angegebene Bedeutung haben,
in Gegenwart von Formaldehyd sowie gegebenenfalls in Gegenwart eines Verdünnungsmittels umsetzt, oder wenn man
(b) Isoxazolidine der Formel (IV),

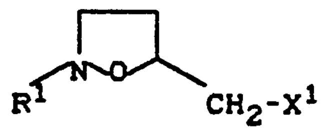

(IV)

in welcher
$R^1$ die oben angegebene Bedeutung hat und
$X^1$ für eine elektronenanziehende Abgangsgruppe steht,
mit Aminen der Formel (V),

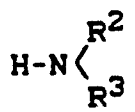

(V)

in welcher
$R^2$ und $R^3$ die oben angegebene Bedeutung haben,
gegebenenfalls in Gegenwart eines Verdünnungsmittels und gegebenenfalls in Gegenwart eines Säurebindemittels sowie gegebenenfalls in Gegenwart eines Katalysators umsetzt oder wenn man
(c) die nach Verfahren (a) oder (b) erhältlichen Aminomethylisoxazolidine der Formel (Ia),

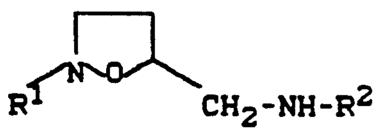

(Ia)

in welcher
$R^1$ und $R^2$ die oben angegebene Bedeutung haben, mit Alkylierungsmitteln der Formel (VI),

$R^{3-1}$-$X^2$ (VI)

in welcher
$R^{3-1}$ für jeweils gegebenenfalls substituiertes Alkyl, Alkenyl oder Cycloalkyl steht und
$X^2$ für eine elektronenanziehende Abgangsgruppe steht,
gegebenenfalls in Gegenwart eines Verdünnungsmittels und gegebenenfalls in Gegenwart eines Säurebindemittels sowie gegebenenfalls in Gegenwart eines Katalysators umsetzt und gegebenenfalls anschließend eine Säure addiert.

Schließlich wurde gefunden, daß die neuen Aminomethylisoxazolidine fungizide Eigenschaften besitzen.

Überraschenderweise zeigen die erfindungsgemäßen Aminomethylisoxazolidine eine erheblich bessere fungizide Wirksamkeit als die aus dem Stand der Tecknik bekannten Isoxazolidine, wie beispielsweise das 2-Methyl-5-(2-pyridyl)-isoxazolidin oder das 5-(3,4-Dichlorbenzyl)-5-ethoxycarbonyl-2-isopropyl-isoxazolidin oder das 5-Benzyl-5-ethoxycarbonyl-2-t-butyl-isoxazolidin, welches chemisch und wirkungsmäßig naheliegende Verbindungen sind.

Die erfindungsgemäßen Aminomethylisoxazolidine sind durch die Formel (I) allgemein definiert. Bevorzugt sind Verbindungen der Formel (I), bei welchen
$R^1$ für jeweils geradkettiges oder verzweigtes Alkyl, Hydroxyalkyl, Fluoralkyl, Alkoxyalkyl oder Fluor alkoxyalkyl mit jeweils 1 bis 18 Kohlenstoffatomen und gegebenenfalls 1 bis 9 Fluoratomen steht, außerdem für gegebenenfalls einfach bis mehrfach, gleich oder verschieden im Arylteil substituiertes Arylalkyl, Aryloxalkyl oder Arylthioalkyl mit jeweils 6 bis 10 Kohlenstoffatomen im Arylteil und 1 bis 10 Kohlenstoffatomen im geradkettigen oder verzweigten Alkylteil steht, wobei als Arylsubstituenten jeweils infrage kommen: Halogen, Cyano, Nitro, jeweils geradkettiges oder verzweigtes Alkyl, Alkoxy, Alkylthio, Halogenalkyl, Halogenalkoxy und Halogenalkylthio mit jeweils 1 bis 6 Kohlenstoffatomen und gegebenenfalls 1 bis 9 gleichen oder verschiedenen Halogenatomen, Amino, jeweils geradkettiges oder verzweigtes Alkylamino, Dialkylamino oder Alkoximinoalkyl mit jeweils 1 bis 4 Kohlenstoffatomen in den einzelnen Alkylteilen;

außerdem für jeweils gegebenenfalls im Cycloalkylteil einfach bis mehrfach, gleich oder verschieden substituiertes Cycloalkylalkyl oder Cycloalkyl mit jeweils 3 bis 10 Kohlenstoffatomen im Cycloalkylteil und gegebenenfalls 1 bis 6 Kohlenstoffatomen im geradkettigen oder verzweigten Alkylteil steht, wobei als Substituenten jeweils infrage kommen: Hydroxy, jeweils geradkettiges oder verzweigtes Alkyl, Alkoxy, Halogenalkyl oder Halogenalkoxy mit jeweils 1 bis 6 Kohlenstoffatomen und gegebenenfalls 1 bis 9 gleichen oder verschiedenen Halogenatomen;

schließlich für jeweils gegebenenfalls einfach bis mehrfach, gleich oder verschieden substituiertes Tetrahydronaphthyl oder Decahydronaphthyl steht, wobei als Substituenten jeweils infrage kommen: Halogen, jeweils geradkettiges oder verzweigtes Alkyl, Alkoxy, Alkylthio, Halogenalkyl, Halogenalkoxy, Halogenalkylthio und Alkoximinoalkyl mit jeweils 1 bis 4 Kohlenstoffatomen und gegebenenfalls 1 bis 9 gleichen oder verschiedenen Halogenatomen und

$R^2$ und $R^3$ unabhängig voneinander jeweils für Wasserstoff, für jeweils geradkettiges oder verzweigtes Alkyl mit 1 bis 8 Kohlenstoffatomen, Hydroxyalkyl mit 2 bis 8 Kohlenstoffatomen oder Alkoxyalkyl mit 3 bis 8 Kohlenstoffatomen, für geradkettiges oder verzweigtes Alkenyl mit 3 bis 8 Kohlenstoffatomen, für jeweils gegebenenfalls einfach bis mehrfach, gleich oder verschieden durch Alkyl mit 1 bis 4 Kohlenstoffatomen und/oder Halogen substituiertes Cycloalkyl mit 3 bis 7 Kohlenstoffatomen oder für jeweils gegebenenfalls im Arylteil einfach bis mehrfach, gleich oder verschieden substituiertes Aralkyl oder Aryl mit 6 bis 10 Kohlenstoffatomen im Arylteil und gegebenenfalls 1 bis 4 Kohlenstoffatomen im geradkettigen oder verzweigten Alkylteil stehen, wobei als Substituenten jeweils infrage kommen: Halogen, Cyano, Nitro, jeweils geradkettiges oder verzweigtes Alkyl, Alkoxy, Alkylthio, Halogenalkyl, Halogenalkoxy, Halogenalkylthio oder Alkoximinoalkyl mit jeweils 1 bis 4 Kohlenstoffatomen in den einzelnen Alkylteilen und gegebenenfalls 1 bis 9 gleichen oder verschiedenen Halogenatomen oder

$R^2$ und $R^3$ gemeinsam mit dem Stickstoffatom, an welches sie gebunden sind, für einen gegebenenfalls einfach bis mehrfach, gleich oder verschieden substituierten gesättigten 5-bis 7-gliedrigen Heterocyclus stehen, der gegebenenfalls weitere Heteroatome, insbesondere Stickstoff, Sauerstoff und/oder Schwefel enthalten kann, wobei als Substituenten infrage kommen: jeweils geradkettiges oder verzweigtes Alkyl oder Hydroxyalkyl mit jeweils 1 bis 4 Kohlenstoffatomen.

Besonders bevorzugt sind Verbindungen der Formel (I), bei welchen

$R^1$ für geradkettiges oder verzweigtes Alkyl mit 1 bis 12 Kohlenstoffatomen steht; außerdem für im Phenylteil gegebenenfalls ein-bis dreifach, gleich oder verschieden substituiertes Phenylalkyl, Phenoxyalkyl oder Phenylthioalkyl mit jeweils 1 bis 8 Kohlenstoffatomen in den jeweiligen geradkettigen oder verzweigten Alkylteilen steht, wobei als Phenylsubstituenten jeweils infrage kommen: Fluor, Chlor, Brom, geradkettiges oder verzweigtes Alkyl mit 1 bis 6 Kohlenstoffatomen, Methoxy, Ethoxy, n-oder i-Propoxy, n-, i-, s-oder t-Butoxy, Methylthio, Trifluormethyl, Trifluormethoxy, Trifluormethylthio oder Methoximinomethyl; außerdem für jeweils gegebenenfalls im Cycloalkylteil ein-bis derifach, gleich oder verschieden substituiertes Cycloalkylalkyl oder Cycloalkyl mit jeweils 5, 6 oder 10 Kohlenstoffatomen im Cycloalkylteil und gegebenenfalls 1 bis 4 Kohlenstoffatomen im Alkylteil steht, wobei als Substituenten jeweils infrage kommen: geradkettiges oder verzweigtes Alkyl mit 1 bis 5 Kohlenstoffatomen; darüberhinaus für gegebenenfalls ein-bis dreifach, gleich oder verschieden substituiertes Decahydronaphthyl steht, wobei als Substituenten genannt seien: geradkettiges oder verzweigtes Alkyl mit 1 bis 5 Kohlenstoffatomen und schließlich für gegebenenfalls ein-bis dreifach, gleich oder verschieden substituiertes Tetrahydronaphthyl steht, wobei als Substituenten infrage kommen: geradkettiges oder verzweigtes Alkyl mit 1 bis 5 Kohlenstoffatomen, Methoxy, Trifluormethyl, Trifluormethoxy oder Chlor,

$R^2$ und $R^3$ unabhängig voneinander jeweils für Wasserstoff, Methyl, Ethyl, n-oder i-Propyl, n-, i-, s-oder t-Butyl, n-oder i-Pentyl, n-oder i-Hexyl, Allyl, n-oder i-Butenyl, n-oder i-Pentenyl, Hydroxypropyl, Methoxyethyl, Ethoxyethyl, Methoxypropyl, Ethoxypropyl, Propoxypropyl, für Cyclopropylmethyl, Dichlorcyclopropylmethyl, für Dichlordimethylcyclopropylmethyl, für Dimethylcyclopropylmethyl oder Cyclohexyl stehen oder

$R^2$ und $R^3$ gemeinsam mit dem Stickstoffatom, an welches sie gebunden sind, für einen gegebenenfalls ein-bis dreifach, gleich oder verschieden substituierten Heterocyclus der Formel

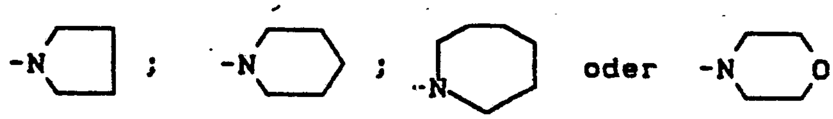

stehen, wobei als Substituenten infrage kommen: Methyl, Ethyl und Hydroxymethyl.

Bevorzugte erfindungsgemäße Verbindungen sind auch Additionsprodukte aus Säuren und denjenigen Aminomethylisoxazolidinen der Formel (I), in denen die Substituenten $R^1$, $R^2$ und $R^3$ die Bedeutung haben,

die bereits vorzugsweise für diese Substituenten genannt wurden.

Zu den Säuren die addiert werden können, gehören vorzugsweise Halogenwasserstoffsäuren, wie z.B. Chlorwasserstoffsäure und Bromwasserstoffsäure, insbesondere Chlorwasserstoffsäure, ferner Phosphorsäure, Salpetersäure, mono-, bi-und trifunktionelle Carbonsäuren und Hydroxycarbonsäuren, wie z.B. Essigsäure, Maleinsäure, Bernsteinsäure, Fumarsäure, Weinsäure, Zitronensäure, Salicylsäure, Sorbinsäure und Milchsäure, ferner Sulfonsäuren, wie p-Toluolsulfonsäure und 1,5-Naphthalindisulfonsäure sowie Saccharin.

Im einzelnen seien außer den bei den Herstellungsbeispielen genannten Verbindungen die folgenden Aminomethylisoxazolidine der allgemeinen Formel (I) genannt:

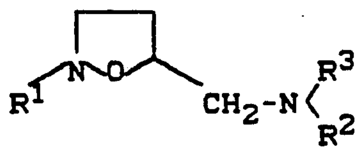 (I)

| $R^1$ | 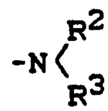 |
|---|---|
| 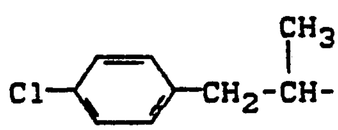 | 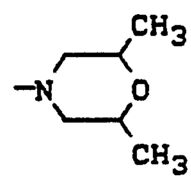 |
| 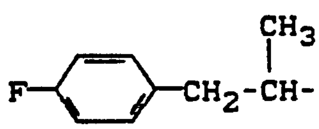 | 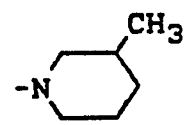 |
| 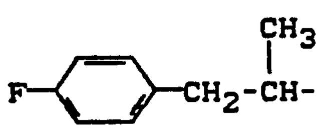 | 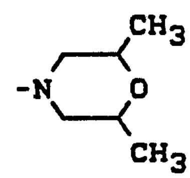 |

| $R^1$ | $-N\langle^{R^2}_{R^3}$ |
|---|---|
| 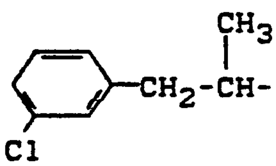 | 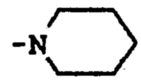 |
| 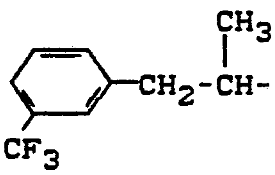 | 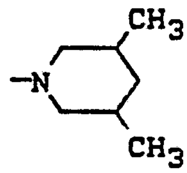 |
| 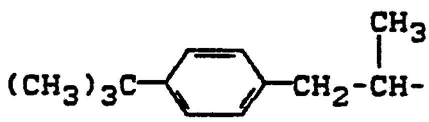 | 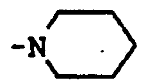 |
| 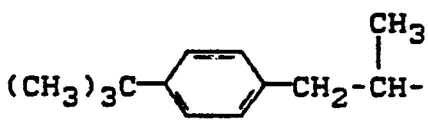 | 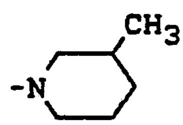 |
| 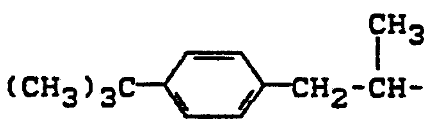 | 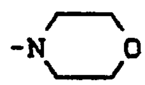 |
| 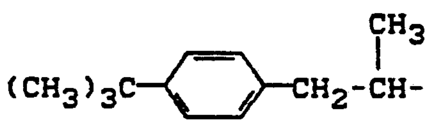 | 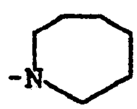 |
| 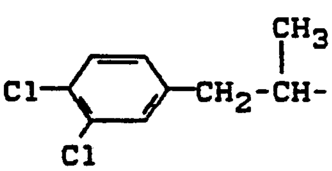 | 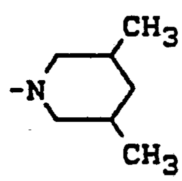 |

| $R^1$ | 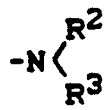 |
| --- | --- |
| 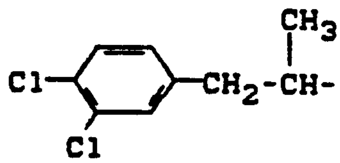 | 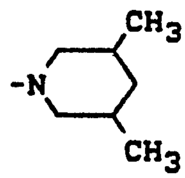 |
| 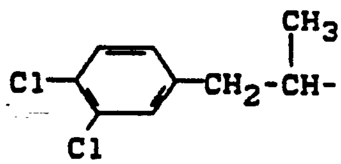 | 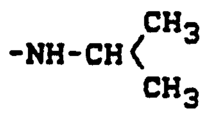 |
| 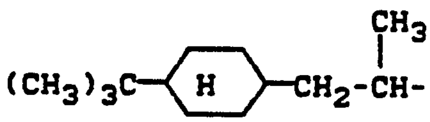 | 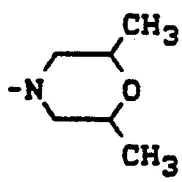 |
| 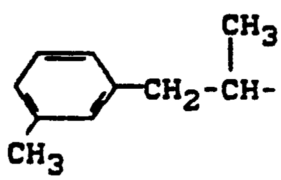 | 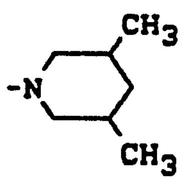 |
| 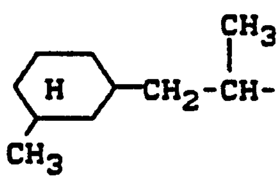 | 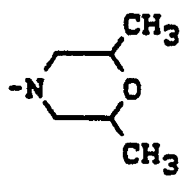 |
| $CH_3$- | 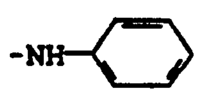 |
| $CH_3$- | 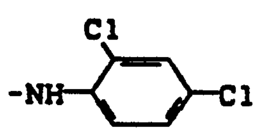 |

| $R^1$ | 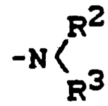 |
|---|---|
| 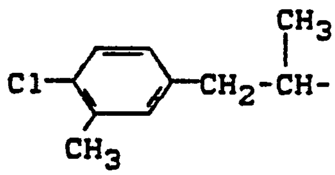 | 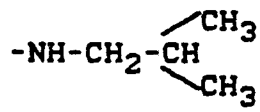 |
| 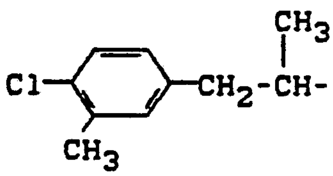 | 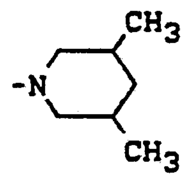 |
| 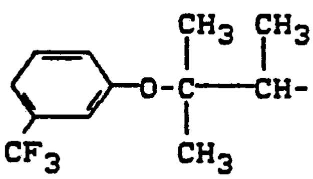 | 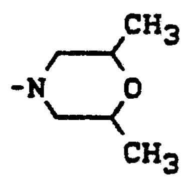 |
| 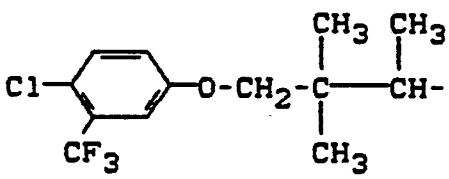 | 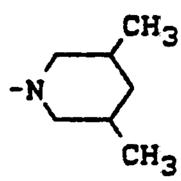 |
| 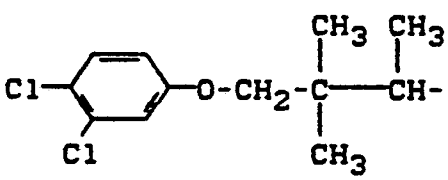 | 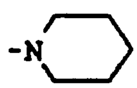 |
| 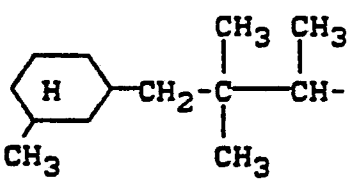 | 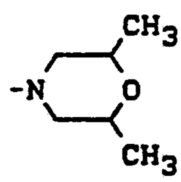 |
| 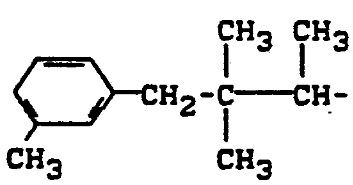 | 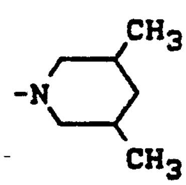 |

| $R^1$ | $-N<{R^2 \atop R^3}$ |
|---|---|
| 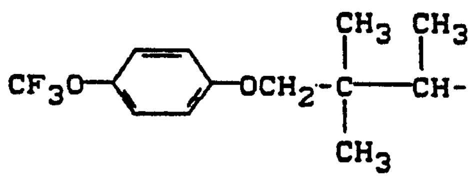 | 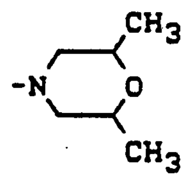 |
| 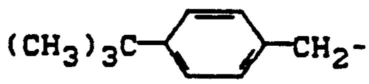 | 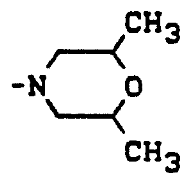 |
| 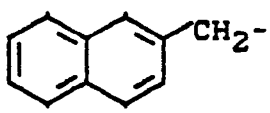 | 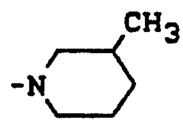 |
| 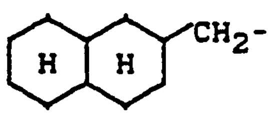 | 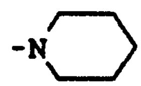 |
| 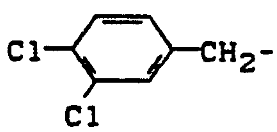 | 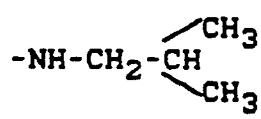 |
| 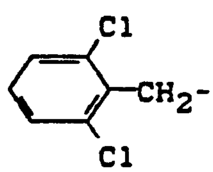 | $-NH-CH_2-CH_2-CH_3$ |
| 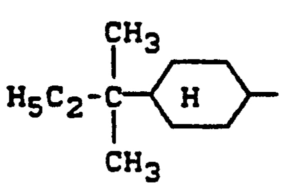 | 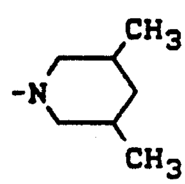 |

| $R^1$ | $-N{<}^{R^2}_{R^3}$ |
|---|---|
| 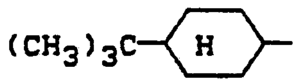 | 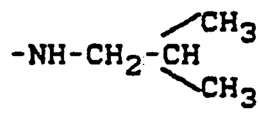 |
| 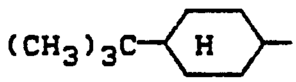 | 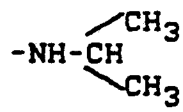 |
| 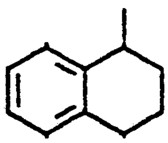 | 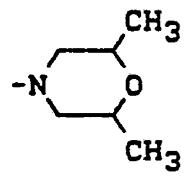 |
| 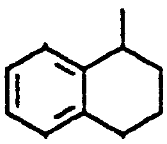 | 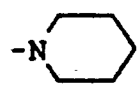 |
| 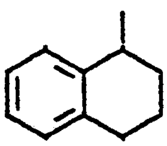 | 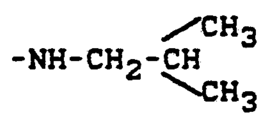 |

Verwendet man beispielsweise N-[3-(3,4-Dichlorphenyl)-2,2-dimethylpropyl]-hydroxylamin, Formaldehyd und N-Allylpiperidin als Ausgangsstoffe, so läßt sich der Reaktionsablauf des erfindungsgemäßen Verfahrens (a) durch das folgende Formelschema darstellen:

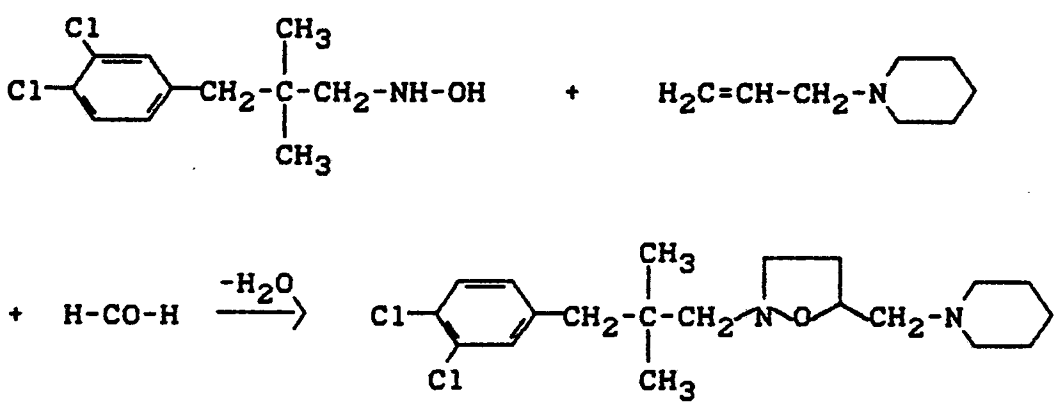


Verwendet man beispielsweise 2-[4-(4-Fluorphenoxy)-3,3-dimethyl-but-2-yl]-5-brommethylisoxazolidin und 3-Methylpiperidin als Ausgangsstoffe, so läßt sich der Reaktionsablauf des erfindungsgemäßen Verfahrens (b) durch das folgende Formelschema darstellen:

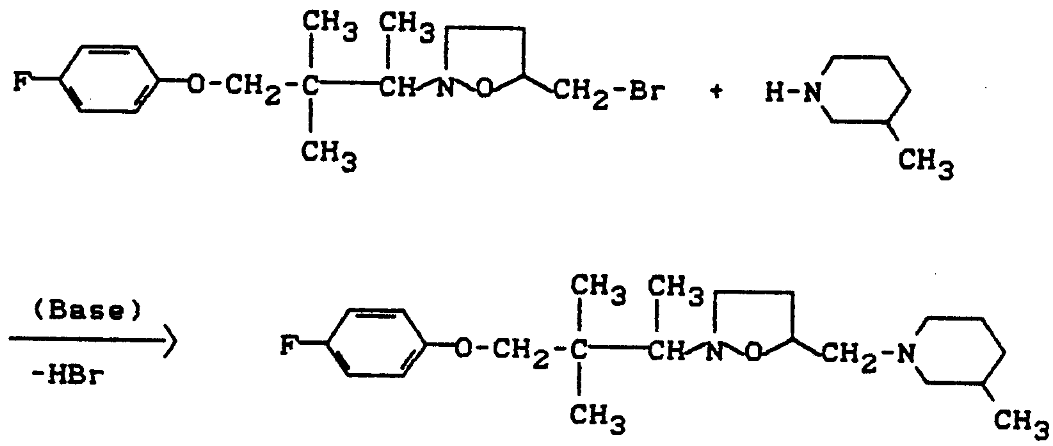


Verwendet man beispielsweise 2-Cyclohexyl-5-aminomethylisoxazolidin und Methyliodid als Ausgangsstoffe, so läßt sich der Reaktionsablauf des erfindungsgemäßen Verfahrens (c) durch das folgende Formelschema darstellen:

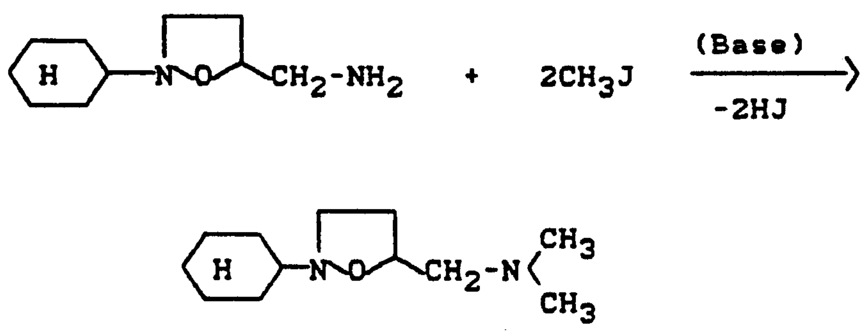


Die zur Durchführung des erfindungsgemäßen Verfahrens (a) als Ausgangsstoffe benötigten Hydroxylamin-Derivate sind durch die Formel (II) allgemein definiert. In dieser Formel (II) steht $R^1$ vorzugsweise für diejenigen Reste, die bereits im Zusammenhang mit der Beschreibung der erfindungsgemäßen Stoffe der Formel (I) als bevorzugt für diesen Substituenten genannt wurden.

Die Hydroxylamin-Derivate der Formel (II) sind größtenteils bekannt (vgl. z.B. Tetrahedron 31, 1531 - 1535 [1975]; Chem. Pharm. Bull. 29, 1615 - 1623 [1981]; DE-OS 23 37 021; Z. Naturforsch. 19 b, 1021 - 1026 [1964]; Chem. Ber. 92, 63-70 [1959]; DE 950 369 [1956]; DE-OS 34 18 395).

Noch nicht bekannt sind Hydroxylamine der Formel (11a),

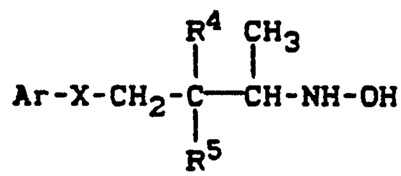 (IIa)

in welcher

Ar für gegebenenfalls substituiertes Phenyl steht, wobei als Substituenten infrage kommen: Halogen, Cyano, Nitro, jeweils geradkettiges oder verzweigtes Alkyl, Alkoxy, Alkylthio, Halogenalkyl, Halogenalkoxy und Halogenalkylthio mit jeweils 1 bis 6 Kohlenstoffatomen und gegebenenfalls 1 bis 9 gleichen oder verschiedenen Halogenatomen, Amino, jeweils geradkettiges oder verzweigtes Alkylamino, Dialkylamino oder Alkoximinoalkyl mit jeweils 1 bis 4 Kohlenstoffatomen in den einzelnen Alkylteilen;

X für Sauerstoff oder Schwefel steht und

$R^4$ und $R^5$ unabhängig voneinander jeweils für Alkyl, insbesondere für Methyl, Ethyl oder i-Propyl stehen.

Man erhält die bekannten ebenso wie die noch nicht bekannten Hydroxylamin-Derivate der Formel (II) in Analogie zu bekannten Verfahren, beispielsweise wenn man Aldehyde oder Ketone der Formel (VII),

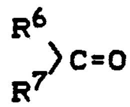 (VII)

in welcher
$R^6$ für gegebenenfalls substituiertes Alkyl steht,
$R^7$ für gegebenenfalls substituiertes Alkyl oder Wasserstoff steht oder
$R^6$ und $R^7$ gemeinsam mit dem Kohlenstoffatom, an dem sie stehen, für gegebenenfalls substituiertes Cycloalkyl, für gegebenenfalls substituiertes Tetrahydronaphthyl oder für gegebenenfalls substituiertes Decahydronaphthyl stehen,
mit Hydroxylaminhydrochlorid gegebenenfalls in Gegenwart einer Base, wie beispielsweise Natriumhydroxid, und gegebenenfalls in Gegenwart eines Verdünnungsmittels, wie beispielsweise Wasser, bei Temperaturen zwischen 10 °C und 100 °C umsetzt, und die so erhältlichen Oxime der Formel (VIII),

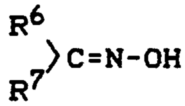 (VIII)

in welcher
$R^6$ und $R^7$ die oben angegebene Bedeutung haben,
in einer 2. Stufe mit Reduktionsmitteln, wie beispielsweise Natrium-cyano-borhydrid, gegebenenfalls in Gegenwart eines Verdünnungsmittels, wie beispielsweise Methanol, bei Temperaturen zwischen 0 °C und 50 °C reduziert (vgl. auch die Herstellungsbeispiele).

Die Ketone und Aldehyde der Formel (VII) sind allgemein bekannte Verbindungen der organischen Chemie. Die Ketone der Formel (VIIa),

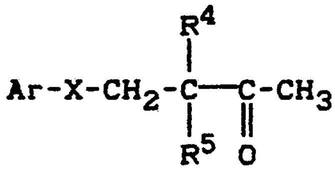 (VIIa)

in welcher
Ar, X, $R^4$ und $R^5$ die oben angegebene Bedeutung haben, die zur Herstellung der noch nicht bekannten Hydroxylamin-Derivate der Formel (IIa) benötigt werden, sind ebenfalls bekannt
(vgl. z.B. DE-OS 32 10 725 oder DE-OS 30 48 266).

Die Aldehyde und Ketone sind durch die Formel (VII) definiert. In ihr stehen bevorzugt
$R^6$ für jeweils geradkettiges oder verzweigtes Alkyl, Hydroxyalkyl, Fluoralkyl, Alkoxyalkyl oder Fluoralkoxyalkyl mit jeweils 1 bis 17 Kohlenstoffatomen und gegebenenfalls 1 bis 9 Fluoratomen, außerdem für gegebenenfalls einfach bis mehrfach, gleich oder verschieden im Arylteil substituiertes Arylalkyl, Aryloxalkyl oder Arylthioalkyl mit jeweils 6 bis 10 Kohlenstoffatomen im Arylteil und 1 bis 10 Kohlenstoffatomen im geradkettigen oder verzweigten Alkylteil, wobei als Arylsubstituenten jeweils infrage kommen: Halogen, Cyano, Nitro, jeweils geradkettiges oder verzweigtes Alkyl, Alkoxy, Alkylthio, Halogenalkyl, Halogenalkoxy und Halogenalkylthio mit jeweils 1 bis 6 Kohlenstoffatomen und gegebenenfalls 1 bis 9 gleichen oder verschiedenen Halogenatomen, Amino, jeweils geradkettiges oder verzweigtes Alkylamino, Dialkylamino oder Alkoximinoalkyl mit jeweils 1 bis 4 Kohlenstoffatomen in den einzelnen Alkylteilen; außerdem für gegebenenfalls im Cycloalkylteil einfach bis mehrfach, gleich oder verschieden substituiertes Cycloalkylalkyl mit 3 bis 10 Kohlenstoffatomen im Cycloalkylteil und gegebenenfalls 1 bis 5 Kohlenstoffatomen im geradkettigen oder verzweigten Alkylteil, wobei als Substituenten jeweils infrage kommen: Hydroxy, jeweils geradkettiges oder verzweigtes Alkyl, Alkoxy, Halogenalkyl oder Halogenalkoxy mit jeweils 1 bis 6 Kohlenstoffatomen und gegebenenfalls 1 bis 9 gleichen oder verschiedenen Halogenatomen;
$R^7$ die Bedeutung von $R^6$ hat oder Wasserstoff ist oder
$R^6$ und $R^7$ gemeinsam mit dem Kohlenstoffatom, an dem sie stehen, einen Cycloalkylrest mit 3 bis 10 Kohlenstoffatomen bilden,
oder einen jeweils gegebenenfalls einfach bis mehrfach, gleich oder verschieden substituierten Tetrahydronaphthyl-oder Decahydronaphthylring bilden, wobei als Substituenten jeweils infrage kommen:

Halogen, jeweils geradkettiges oder verzweigtes Alkyl, Alkoxy, Alkylthio, Halogenalkyl, Halogenalkoxy, Halogenalkylthio und Alkoximinoalkyl mit jeweils 1 bis 4 Kohlenstoffatomen und gegebenenfalls 1 bis 9 gleichen oder verschiedenen Halogenatomen.

Besonders bevorzugt sind Verbindungen der Formel (VII), bei welchen

$R^6$ für geradkettiges oder verzweigtes Alkyl mit 1 bis 11 Kohlenstoffatomen steht; außerden für im Phenylteil gegebenenfalls ein-bis dreifach, gleich oder verschieden substituiertes Phenylalkyl, Phenoxyalkyl oder Phenylthioalkyl mit jeweils 1 bis 7 Kohlenstoffatomen in den jeweiligen geradkettigen oder verzweigten Alkylteilen steht, wobei als Phenylsubstituenten jeweils infrage kommen: Fluor, Chlor, Brom, geradkettiges oder verzweigtes Alkyl mit 1 bis 6 Kohlenstoffatomen, Methoxy, Ethoxy, n-oder i-Propoxy, n-, i-, s-oder t-Butoxy, Methylthio, Trifluormethyl, Trifluormethoxy, Trifluormethylthio oder Methoximinomethyl; außerdem für gegebenenfalls im Cycloalkylteil ein-bis dreifach, gleich oder verschieden substituiertes Cycloalkylalkyl mit 5, 6 oder 10 Kohlenstoffatomen im Cycloalkylteil und gegebenenfalls 1 bis 3 Kohlenstoffatomen im Alkylteil steht, wobei als Substituenten jeweils infrage kommen: geradkettiges oder verzweigtes Alkyl mit 1 bis 5 Kohlenstoffatomen;

$R^7$ die bei $R^6$ angegebene Bedeutung hat oder für Wasserstoff steht oder

$R^6$ und $R^7$ gemeinsam mit dem Kohlenstoffatom, an dem sie stehen, einen Cycloalkylrest mit 5, 6 oder 10 Kohlenstoffatomen bilden, der ein-bis dreifach, gleich oder verschieden substituiert sein kann durch geradkettiges oder verzweigtes Alkyl mit 1 bis 5 Kohlenstoffatomen; darüberhinaus einen gegebenenfalls ein-bis dreifach, gleich oder verschieden substituierten Decahydronaphthylring bilden, wobei als Substituenten genannt seien: geradkettiges oder verzweigtes Alkyl mit 1 bis 5 Kohlenstoffatomen; und schließlich einen gegebenenfalls ein-bis dreifach, gleich oder verschieden substituierten Tetrahydronaphthylring bilden, wobei als Substituenten infrage kommen: geradkettiges oder verzweigtes Alkyl mit 1 bis 5 Kohlenstoffatomen, Methoxy, Trifluormethyl, Trifluormethoxy oder Chlor.

Die zur Durchführung des erfindungsgemäßen Verfahrens (a) weiterhin als Ausgangsstoffe benötigten Allylamine sind durch die Formel (III) allgemein definiert. In dieser Formel (III) stehen $R^2$ und $R^3$ vorzugsweise für diejenigen Reste, die bereits im Zusammenhang mit der Beschreibung der erfindungsgemäßen Stoffe der Formel (I) für diese Substituenten genannt wurden.

Die Allylamine der Formel (III) sind ebenfalls allgemein bekannte Verbindungen der organischen Chemie.

Die zur Durchführung des erfindungsgemäßen Verfahrens (b) als Ausgangsstoffe benötigten Isoxazolidine sind durch die Formel (IV) allgemein definiert. In dieser Formel (IV) steht $R^1$ vorzugsweise für diejenigen Reste, die bereits im Zusammenhang mit der Beschreibung der erfindungsgemäßen Stoffe der Formel (I) als bevorzugt für diesen Substituenten genannt wurden.

$X^1$ steht vorzugsweise für Halogen, insbesondere für Chlor oder Brom, oder für gegebenenfalls substituiertes Alkylsulfonyloxy oder Arylsulfonyloxy, wie beispielsweise Methansulfonyloxy oder p-Toluolsulfonyloxy.

Die Isoxazolidine der Formel (IV) sind noch nicht bekannt. Man erhält sie in Analogie zum erfindungsgemäßen Verfahren (a), wenn man Hydroxylamin-Derivate der Formel (II),

$R^1$-NH-OH (II)

in welcher

$R^1$ die oben angegebenen Bedeutung hat,

mit Allylhalogeniden oder Allylakohol der Formel (IX),

$CH_2=CH-CH_2-X^3$ (IX)

in welcher

$X^3$ für Hydroxy oder Halogen, insbesondere für Hydroxy, Chlor oder Brom steht,

in Gegenwart von Formaldehyd sowie gegebenenfalls in Gegenwart eines Verdünnungsmittel, wie beispielsweise Methanol oder Tetrachlorkohlenstoff, bei Temperaturen zwischen 20 °C und 80 °C umsetzt und in den Fällen, wo $X^3$ für Hydroxy steht, die so erhältlichen Hydroxymethylisoxazolidine der Formel (X),

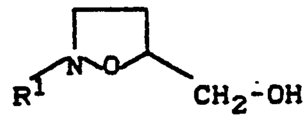

(X)

in welcher

$R^1$ die oben angegebene Bedeutung hat,

in einer 2. Stufe mit Sulfonylhalogeniden der Formel (XI),

$X^4$-$SO_2$-$R^4$ (XI)

in welcher

$X^4$ für Halogen, insbesondere für Chlor oder Brom steht und

$R^4$ für gegebenenfalls substituiertes Alkyl oder Aryl, wie beispielsweise Methyl oder p-Tolyl steht,

gegebenenfalls in Gegenwart eines Verdünnungsmittels, wie beispielsweise Dichlormethan und gegebenenfalls in Gegenwart eines basischen Reaktionshilfsmittels, wie beispielsweise Triethylamin bei Temperaturen zwischen 10 °C und 60 °C umsetzt.

Die Allylhalogenide bzw. der Allylalkohol der Formel (IX) und die Sulfonylhalogenide der Formel (XI) sind allgemein bekannte Verbindungen der organischen Chemie.

Die zur Durchführung des erfindungsgemäßen Verfahrens (b) weiterhin als Ausgangsstoffe benötigten Amine sind durch die Formel (V) allgemein definiert. In dieser Formel (V) stehen $R^2$ und $R^3$ für diejenigen Reste, die bereits im Zusammenhang mit der Beschreibung der erfindungsgemäßen Stoffe der Formel (I) für diese Substituenten genannt wurden.

Die Amine der Formel (V) sind ebenfalls allgemein bekannte Verbindungen der organischen Chemie.

Die zur Durchführung des erfindungsgemäßen Verfahrens (c) als Ausgangsstoffe benötigten Aminomethylisoxazolidine sind durch die Formel (Ia) allgemein definiert. In dieser Formel (Ia) stehen $R^1$ und $R^2$ für diejenigen Reste die bereits im Zusammenhang mit der Beschreibung der erfindungsgemäßen Stoffe der Formel (I) für diese Substituenten genannt wurden.

Die Aminomethylisoxazolidine der Formel (Ia) sind erfindungsgemäße Verbindungen und erhältlich mit Hilfe der erfindungsgemäßen Verfahren (a) und (b).

Die zur Durchführung des erfindungsgemäßen Verfahrens (c) weiterhin als Ausgangsstoffe benötigten Alkylierungsmittel sind durch die Formel (VI) allgemein definiert. In dieser Formel (VI) steht $R^{3-1}$ vorzugsweise für geradkettiges oder verzweigtes Alkyl mit 1 bis 8 Kohlenstoffatomen, für Hydroxyalkyl mit 2 bis 8 Kohlenstoffatomen oder Alkoxyalkyl mit 3 bis 8 Kohlenstoffatomen, für geradkettiges oder verzweigtes Alkenyl mit 3 bis 8 Kohlenstoffatomen, für jeweils gegebenenfalls einfach bis mehrfach, gleich oder verschieden durch Alkyl mit 1 bis 4 Kohlenstoffatomen und/oder Halogen substituiertes Cycloalkyl oder Cycloalkylalkyl mit jeweils 3 bis 7 Kohlenstoffatomen in Cycloalkylteil und gegebenenfalls 1 bis 3 Kohlenstoffatomen im Alkylteil oder für gegebenenfalls im Arylteil einfach bis mehrfach, gleich oder verschieden substituiertes Aralkyl mit 6 bis 10 Kohlenstoffatomen im Arylteil und 1 bis 4 Kohlenstoffatomen im geradkettigen oder verzweigten Alkylteil, wobei als Substituenten genannt seien: Halogen, Cyano, Nitro, jeweils geradkettiges oder verzweigtes Alkyl, Alkoxy, Alkylthio, Halogenalkyl, Halogenalkoxy, Halogenalkylthio oder Alkoximinoalkyl mit jeweils 1 bis 4 Kohlenstoffatomen in den einzelnen Alkylteilen und gegebenenfalls 1 bis 9 gleichen oder verschiedenen Halogenatomen. $R^{3-1}$ steht insbesondere für Methyl, Ethyl, n-oder i-Propyl, n-, i-, s-oder t-Butyl, n-oder i-Pentyl, n-oder i-Hexyl, Allyl, n-oder i-Butenyl, n-oder i-Pentenyl, Hydroxypropyl, Methoxyethyl, Ethoxyethyl, Methoxpropyl, Ethoxypropyl, Propoxypropyl, für Cyclopropylmethyl, Dichlorcyclopropylmethyl, Dimethylcyclopropylmethyl oder Dichlordimethylcyclopropylmethyl. $X^2$ steht vorzugsweise für Halogen, insbesondere für Chlor oder Brom oder für gegebenenfalls substituiertes Alkylsulfonyloxy oder Arylsulfonyloxy, wie beispielsweise Methansulfonyloxy oder p-Toluolsulfonyloxy. Die Alkylierungsmittel der Formel (VI) sind ebenfalls allgemein bekannte Verbindungen der organischen Chemie.

Als Verdünnungsmittel zur Durchführung des erfindungsgemäßen Verfahrens (a) kommen inerte organische Lösungsmittel oder deren Gemische mit Wasser infrage.

Hierzu gehören insbesondere aliphatische oder aromatische, gegebenenfalls halogenierte Kohlenwasserstoffe, wie beispielsweise Cyclohexan, Dichlormethan, Chloroform, Tetrachlorkohlenstoff, Ether, wie Dioxan, Tetrahydrofuran oder Ethylenglykoldimethyl-oder -diethylether, Amide, wie Dimethylformamid, Dimethylacetamid, N-Methylformanilid, N-Methylpyrrolidon oder Hexamethylphosphorsäuretriamid, Sulfoxide, wie Dimethylsulfoxid oder Alkohole, wie Methanol, Ethanol oder Propanol und deren Gemische mit Wasser.

Die Reaktionstemperaturen können bei der Durchführung des erfindungsgemäßen Verfahrens (a) in einem größeren Bereich variiert werden. Im allgemeinen arbeitet man bei Temperaturen zwischen 20 °C und 150 °C, vorzugsweise bei Temperaturen zwischen 40 °C und 140 °C.

Zur Durchführung des erfindungsgemäßen Verfahrens (a) setzt man pro Mol an Hydroxylamin-Derivat der Formel (II) im allgemeinen 0,8 bis 1,3 Mol, vorzugsweise äquimolare Mengen an Allylamin der Formel (III) und 1,0 bis 5,0 Mol an Formaldehyd ein.

Es ist auch möglich das Hydroxylamin-Derivate der Formel (II) in Form eines Hydrohalogenid-Salzes, insbesondere eines Hydrochlorides einzusetzen. In diesem Fall ist es zweckmäßig eine äquimolare Menge eines geeigneten Säurebindemittels, wie beispielsweise Natriumhydroxid, Natriumcarbonat, Kaliumcarbonat oder Triethylamin zuzusetzen. Auch ein entsprechender Überschuß an Allylamin der Formel (III) als

Säurebindemittel ist möglich. Daneben ist es ebenfalls möglich den Formaldehyd als wäßrige Formalinlösung einzusetzen.

Die Reaktionsdurchführung, Aufarbeitung und Isolierung der Reaktionsprodukte der Formel (I) erfolgt mit Hilfe üblicher Methoden in Analogie zu bekannten Verfahren (vgl. auch DE-OS 34 18 395 sowie die Herstellungsbeispiele).

Als Verdünnungsmittel zur Durchführung der erfindungsgemäßen Verfahren (b) und (c) kommen inerte organische Lösungsmittel oder wäßrige Systeme infrage.

Hierzu gehören insbesondere aliphatische oder aroma tische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chlororform, Tetrachlorkohlenstoff, Ether, wie Diethylether, Dioxan, Tetrahydrofuran oder Ethylenglykoldimethyl oder -diethylether, Ketone, wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Sulfoxide, wie Dimethylsulfoxid.

Die erfindungsgemäßen Verfahren (b) und (c) können gegebenenfalls auch in einem Zweiphasensystem, wie beispielsweise Wasser/Toluol oder Wasser/Dichlormethan, gegebenenfalls in Gegenwart eines Phasentransferkatalysators, durchgeführt werden. Als Beispiele für solche Katalysatoren seien genannt: Tetrabutylammoniumiodid, Tetrabutylammoniumbromid, Tributyl-methylphosphoniumbromid, Trimethyl-$C_{13}$/$C_{15}$-alkylammoniumchlorid, Dibenzyldimethyl-ammoniummethylsulfat, Dimethyl-$C_{12}$/$C_{14}$-alkyl-benzylammoniumchlorid, Tetrabutylammoniumhydroxid, 15-Krone-5, 18-Krone-6, Triethylbenzylammoniumchlorid, Trimethylbenzylammoniumchlorid. Es ist auch möglich die erfindungsgemäßen Verfahren (b) und (c) ohne Zusatz eines Lösungsmittels durchzuführen.

Als Säurebindemittel zur Durchführung der erfindungsgemäßen Verfahren (b) und (c) kommen alle üblicherweise verwendbaren anorganischen und organischen Basen in frage. Vorzugsweise verwendet man Alkalimetallhydride, -hydroxide, -amide, -carbonate oder -hydrogencarbonate, wie beispielsweise Natriumhydrid, Natriumamid, Natriumhydroxid, Natriumcarbonat oder Natriumhydrogencarbonat oder auch tertiäre Amine, wie beispielweise Triethylamin, N,N-Dimethylanilin, Pyridin, 4-(N,N-Dimethylamino)-pyridin, Diazabicyclooctan (DABCO), Diazabicyclononen (DBN) oder Diazabicycloundecen (DBU).

Es ist auch möglich, die als Reaktionsteilnehmer verwendeten Amine der Formeln (V) bzw (Ia) in entsprechendem Überschuß gleichzeitig als Säurebindemittel einzusetzen.

Die Reaktionstemperaturen können bei der Durchführung der erfindungsgemäßen Verfahren (b) und (c) in einem größeren Bereich variiert werden. Im allgemeinen arbeitet man bei Temperaturen zwischen - 20 °C und + 150 °C, vorzugsweise bei Temperaturen zwischen 0 °C und +120 °C.

Zur Durchführung des erfindungsgemäßen Verfahrens (b) setzt man pro Mol an Isoxazolidin der Formel (IV) im allgemeinen 1,0 bis 3,0 Mol, vorzugsweise 1,0 bis 1,5 Mol an Amin der Formel (V) und gegebenenfalls 1,0 bis 3,0 Mol, vorzugsweise 1,0 bis 1,5 Mol an Säurebindemittel, sowie gegebenenfalls 0,1 bis 1,0 Mol an Phasentransferkatalysator ein.

Zur Durchführung des erfindungsgemäßen Verfahrens (c) setzt man pro Mol an Aminomethylisoxazolidin der Formel (Ia) im allgemeinen 1,0 bis 5,0 Mol, vorzugsweise 1,0, bis 2,0 Mol an Alkylierungsmittel und 1,0 bis 5,0 Mol, vorzugsweise 1,0 bis 2,0 Mol an Säurebindemittel, sowie gegebenenfalls 0,1 bis 1,0 Mol an Phasentransferkatalysator ein.

Die Reaktionsdurchführung, Aufarbeitung und Isolierung der Reaktionsprodukte der Formel (I) erfolgt in beiden Fällen nach üblichen Methoden.

Zur Herstellung von Säureadditionssalzen der Verbindungen der Formel (I) kommen vorzugsweise folgende Säuren infrage: Halogenwasserstoffsäuren, wie z.B. Chlorwasserstoffsäure und Bromwasserstoffsäure, insbesondere Chlorwasserstoffsäure, ferner Phosphorsäure, Salpetersäure, Schwefelsäure, mono-, bi-und trifunktionelle Carbonsäuren und Hydroxycarbonsäuren, wie z.B. Essigsäure, Maleinsäure, Bernsteinsäure, Fumarsäure, Weinsäure, Zitronensäure, Salicylsäure, Sorbinsäure, Milchsäure, sowie Sulfonsäuren, wie z.B. p-Toluolsulfonsäure und 1,5-Naphthalindisulfonsäure sowie Saccharin.

Die Säureadditionssalze der Verbindungen der Formel (I) können in einfacher Weise nach üblichen Salzbildungsmethoden, wie z.B. durch Lösen einer Verbindung der Formel (I) in einem geeigneten inerten Lösungsmittel und Hinzufügen der Säure, wie z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Die erfindungsgemäßen Wirkstoffe weisen eine starke Wirkung gegen Schädlinge auf und können zur Bekämpfung von unerwünschten Schadorganismen praktisch eingesetzt werden. Die Wirkstoffe sind für den Gebrauch u.a. als Pflanzenschutzmittel insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erregervon pilzlichen Erkrankungen, die unter die oben aufgezählten Oberbegriffe fallen, genannt:
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz-und Saatgut, und des Bodens.

Dabei zeigen die erfindungsgemäßen Wirkstoffe eine besonders breite fungizide Wirksamkeit und lassen sich mit besonders gutem Erfolg zur Bekämpfung von Getreidekrankheiten, wie beispielsweise gegen den Erreger des echten Gerstenmehltaus (Erysiphe graminis) oder gegen den Erreger der Blattfleckenkrankheit der Gerste (Pyrenophora teres), gegen Rosterreger oder gegen den Erreger der Blattfleckenkrankheit am Reis (Pyricularia oryzae) einsetzen. Daneben zeigen die erfindungsgemäßen Wirkstoffe auch eine insektizide und akarizide Wirksamkeit.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Wirkstoff-imprägnierte Natur-und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt-und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse

Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder -schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo-und Metallphthalocyaninfarbstoff und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe können als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 % am Wirkungsort erforderlich.

## Herstellungsbeispiele

### Beispiel 1 :

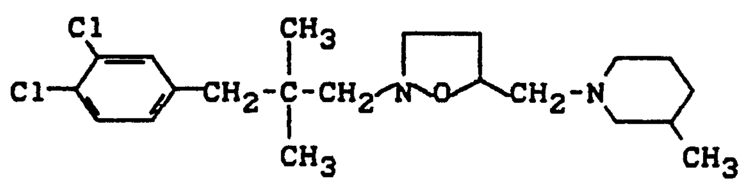

(Verfahren a)

Zu einer Mischung aus 10,8 g (0,038 Mol) 3-(3,4-Dichlorphenyl)-2,2-dimethylpropylhydroxylamin Hydrochlorid und 4 g (0,038 Mol) Formalin (35 proz. Formaldehyd in Wasser) in 100 ml Methanol gibt man 4 g (0,029 Mol) Kaliumcarbonat in 20 ml Methanol, filtriert ausgefallenes Kaliumchlorid ab und gibt das Filtrat bei 100 °C zu einer Lösung aus 11 g (0,076 Mol) 3-Methyl-1-allylpiperidin in 200 ml Toluol, so daß das Methanol langsam abdestilliert. Wenn alles Methanol entfernt ist, erhitzt man für 48 Stunden auf Rückflußtemperatur und entfernt dabei das Wasser über einen Wasserabscheider. Die erkaltete Reaktionsmischung wird zweimal mit Wasser gewaschen, über Magnesiumsulfat getrocknet und im Vakuum

eingeengt. Der Rückstand wird chromatographisch (Kieselgel ; Laufmittel : Petrolether/Essigester 3:1) gereinigt.

Man erhält 4 g (26 % der Theorie) an 2-[3-(3,4-Dichlorphenyl)-2,2-dimethylprop-1-yl]-5-(3-methylpiperidin-1-yl-methyl)-isoxazolidin vom Brechungsindex $n_D^{20}$ 1.5267.

Herstellung der Ausgangsverbindung:

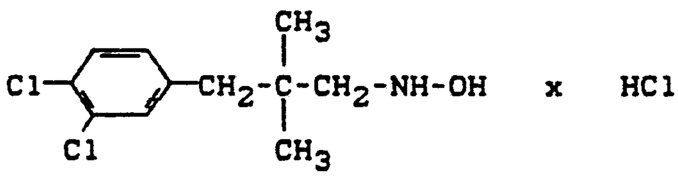

Zu einer Lösung von 95 g (0,39 Mol) 3-(3,4-Dichlorphenyl)-2,2-dimethyl-propionaldoxim in 1 l Methanol die mit Chlorwasserstoffsäure gegen Bromkresolgrün als Indikator auf pH 3 eingestellt wurde, gibt man portionsweise 26 g (0,39 Mol) Natriumcyanoborhydrid, wobei der pH-Wert durch gleichzeitige Zugabe von Chlorwasserstoffsäure zwischen 3 und 5 gehalten wird. Nach beendeter Zugabe rührt man 16 Stunden bei Raumtemperatur, bringt die Mischung dann durch weitere Zugabe von Chlorwasserstoffsäure auf pH 1 und engt im Vakuum ein. Der Rückstand wird in Chloroform/Wasser aufgenommen, die Chloroformphase abgetrennt, mit Wasser gewaschen, über Magnesiumsulfat getrocknet und im Vakuum von Lösungsmittel befreit.

Man erhält 77 g (70 % der Theorie) an 3-(3,4-Dichlorphenyl)-2,2-dimethylpropylhydroxylamin Hydrochlorid, welches ohne Reinigung weiter umgesetzt wird.

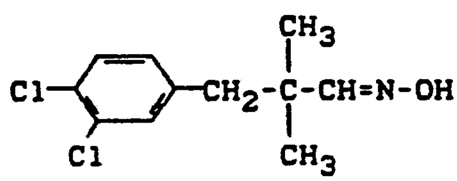

Zu einer Mischung aus 147 g (0,63 Mol) 2-(3,4-Dichlorphenyl)-2,2-dimethylpropionaldehyd und 100 g (2,5 Mol) Natriumhydroxid in 800 ml Wasser gibt man portionsweise 46 g (0,66 Mol) Hydroxylaminhydrochlorid. Nach beendeter Zugabe rührt man 16 Stunden bei Raumtemperatur nach und neutralisiert dann durch Zugabe von Trockeneis. Der ausgefallene Niederschlag wird abgesaugt, mit Wasser gewaschen und getrocknet.

Man erhält 142 g (92 % der Theorie) an 3-(3,4-Dichlorphenyl)-2,2-dimethyl-propionaldoxim vom Schmelzpunkt 84 °C.

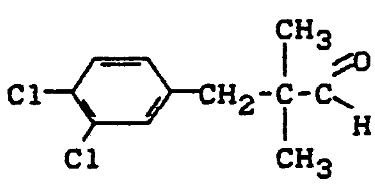

Zu einer Mischung aus 42 g (1,05 Mol) Natriumhydroxid und 3 g Tetrabutylammoniumiodid in 100 ml Wasser und 100 ml Toluol tropft man bei 80 °C langsam unter heftigem Rühren innerhalb von 5 Stunden ein Gemisch aus 68,5 g (0,35 Mol) 3,4-Dichlorbenzylchlorid und 36 g (0,5 Mol) Isobutyraldehyd. Nach beendeter Zugabe rührt man weitere 3 Stunden bei 80 °C, gibt dann 200 ml Toluol zu, wäscht die organische Phase dreimal mit je 100 ml Wasser, trocknet über Natriumsulfat, engt im Vakuum ein und destilliert den Rückstand im Hochvakuum.

Man erhält 57,9g (73 % der Theorie) an 2-(3,4-Dichlorphenyl)-2,2-dimethyl-propionaldehyd von Siedepunkt 92°C/0,13 mbar.

Beispiel 2:

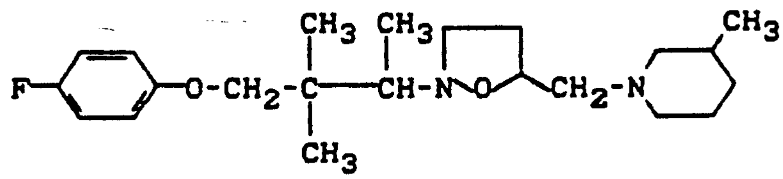

(Verfahren b)

Eine Mischung aus 7 g (0,02 Mol) 5-Brommethyl-2-[4-(4-fluorphenoxy)-3,3-dimethyl-but-2-yl]-isoxazolidin und 4 g (0,04 Mol) 3-Methylpiperidin wird 16 Stunden bei 130 °C gerührt. Zur Aufarbeitung nimmt man die erkaltete Reaktionsmischung in Dichlormethan auf, wäscht mit Wasser, trocknet über Natriumsulfat, engt im Vakuum ein und reinigt das Produkt chromatographisch (Kieselgel; Laufmittel : Petrolether/Essigester 6:1).

Man erhält 4g (51 % der Theorie) an 2-[4-(4-Fluorphenoxy)-3,3-dimethyl-but-2-yl]-5-(3-methylpiperidin-1-yl-methyl)-isoxazolidin vom Brechungsindex $n_D^{20}$ 1.5070.

Herstellung der Ausgangsverbindungen

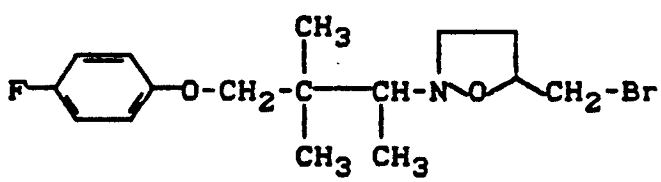

Zu einer Lösung von 26,3 g (0,1 Mol) 4-(4-Fluorphenoxy)-3,3-dimethyl-2-butyl-hydroxylamin Hydrochlorid in 80 ml Methanol gibt man 6,72 g (0,12 Mol) Kaliumhydroxid in 80 ml Methanol, filtriert ausgefallenes Kaliumchlorid ab und versetzt das Filtrat mit 10,3 g (0,12 Mol) Formalin (35prozentige wässrige Formaldehyd-Lösung). Diese Mischung tropft man unter Rühren bei 70 °C zu einer Lösung von 12,1 g (0,1 Mol) Allylbromid in 100 ml Tetrachlorkohlenstoff, die mit 10 g gepulvertem Molekularsieb versetzt wurde, wobei das Methanol langsam abdestilliert. Nach beendeter Zugabe und destillativer Entfernung des Methanoles setzt man 4 ml (0,04 Mol) Allylbromid in 50 ml Tetrachlorkohlenstoff zu und rührt weitere 16 Stunden bei Rückflußtemperatur. Zur Aufarbeitung wird die erkaltete Reaktionsmischung mit Wasser gewaschen, über Natriumsulfat getrocknet und im Vakuum eingeengt.

Zur Reinigung wird über Kieselgel mit Petrolether/Essigester 4 : 1 chromatographiert.

Man erhält 7 g (20 % der Theorie) an 5-Brommethyl-2-[4-(4-fluorphenoxy)-3,3-dimethyl-but-2-yl]-isoxazolidin als Öl. $^1$H-NMR*) : $\delta$ = 4,0-4,3 (m,1H), 3,1-4,0 (m,4H) ppm.

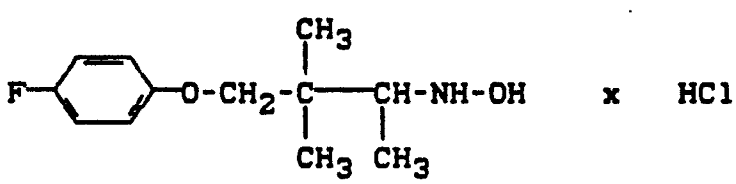

Zu einer Lösung von 66,8 g (0,29 Mol) 2-Hydroximino-3,3-dimethyl-4-(4-fluorphenoxy)-butan in 500 ml Methanol, die mit konzentrierter Salzsäure gegen Bromkresolgrün auf pH 3 gehalten wird, gibt man portionsweise 19,6 g (0,29 Mol) Natriumcyanoborhydrid (95 %). Nach beendeter Zugabe wird für 3 weitere Stunden der pH-Wert mit Salzsäure auf 3 gehalten und dann 16 Stunden bei Raumtemperatur nachgerührt. Die Reaktionsmischung wird auf pH 1 angesäuert und im Vakuum eingeengt. Der Rückstand wird in Chloroform/Wasser aufgenommen, die organische Phase abgetrennt, über Natriumsulfat getrocknet und das Lösungsmitttel im Vakuum abdestilliert. Man erhält 52,3 g (67,1 % der Theorie) an 4-(4-Fluorphenoxy)-3,3-dimethyl-2-butyl-hydroxylamin Hydrochlorid vom Schmelzpunkt 99 °C.

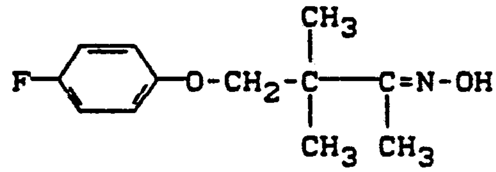

Zu einer Mischung aus 84 g (0,4 Mol) 3,3-Dimethyl-4-(4-fluorphenoxy)-butan-2-on in 200 ml Ethanol und 28,3 g (0,41 Mol) Hydroxylamin Hydrochlorid in 100 ml Wasser tropft man bei 50 °C eine Lösung von 40 g Natriumhydroxid in 80 ml Wasser und läßt 16 Stunden bei 60 °C nachreagieren. Die Reaktionsmischung wird abgekühlt und mit Trockeneis neutralisiert. Der ausgefallene Feststoff wird abgesaugt und getrocknet. Man erhält 69 g (76,6 % der Theorie) 2-Hydroximino-3,3-dimethyl-4-(4-fluorphenoxy)-butan vom Schmelzpunkt 65 °C - 66 °C.

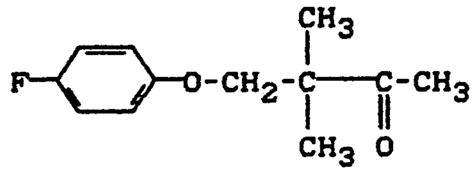

Zu einer Mischung aus 112 g (1 Mol) 4-Fluorphenol und 56 g (1 Mol) Kaliumhydroxid in 30 ml Wasser tropft man bei 115 °C 200 g (1 Mol) 4-Methansulfonyloxy-3,3-dimethyl-butan-2-on (vgl. z.B. DE-OS 28 43 767 oder DE-OS 29 18 894). Nach beendeter Zugabe läßt man 20 Stunden bei 110 °C -115 °C nachreagieren, kühlt ab, verdünnt mit 1000 ml Essigester, wäscht mit 300 ml Wasser, zwei-bis dreimal mit verdünnter wässriger Natronlauge und noch weitere zweimal mit Wasser, trocknet die organische Phase über Natriumsulfat, engt im Vakuum ein und destilliert den Rückstand im Hochvakuum.
Man erhält 159,6 g (76 % der Theorie) an 4-(4-Fluorphenoxy)-3,3-dimethyl-butan-2-on vom Siedepunkt Kp. 90 °C/0,1 mbar.

In entsprechender Weise und gemäß den allgemeinen Angaben zur Herstellung erhält man die folgenden Aminomethylisoxazolidine der allgemeinen Formel (I):

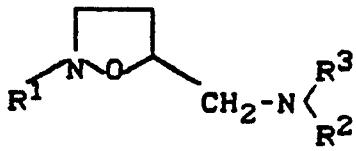 (I)

| Nr. | | $-N<^{R^2}_{R^3}$ | physikalische Daten |
|---|---|---|---|
| 3 | 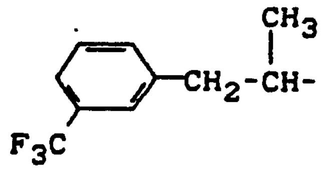 | 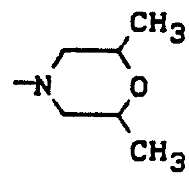 | $n_D^{20}$ 1.4825 |
| 4 | 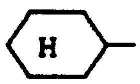 | 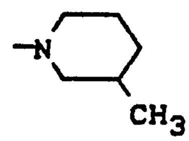 | Kp. 110 °C/ 0,04 mbar |
| 5 | 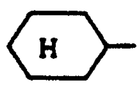 | 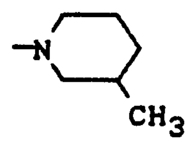 | $n_D^{20}$ 1.5182 |
| | | | (x 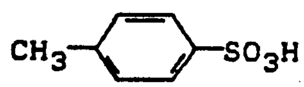) |
| 6 | 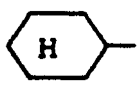 | 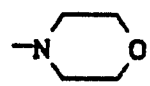 | $n_D^{20}$ 1.4984 |
| 7 | 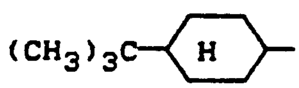 | 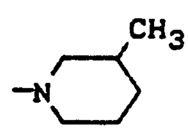 | $n_D^{20}$ 1.4903 |
| 8 | 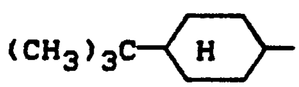 | 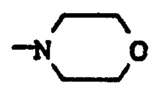 | $n_D^{20}$ 1.4931 |

| Bsp. Nr. | $R^1$ | $-N\langle^{R^2}_{R^3}$ | physikalische Daten |
|---|---|---|---|
| 9 | 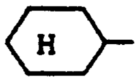 | 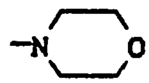 | $^1H$-NMR*): 3.8-4.0; 3.1-3.6 (x 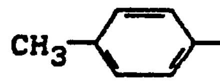) |
| 10 | 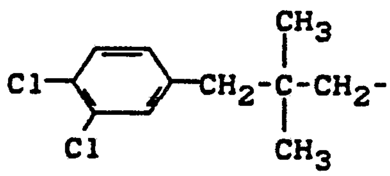 | 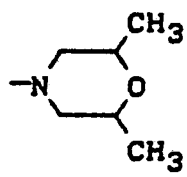 | $n_D^{20}$ 1.5253 |
| 11 | 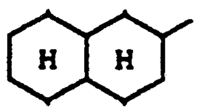 | 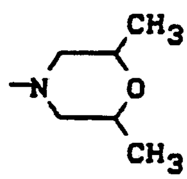 | $n_D^{20}$ 1.5021 |
| 12 | 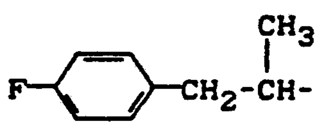 | 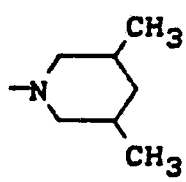 | $n_D^{20}$ 1.5033 |
| 13 | 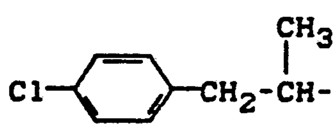 | 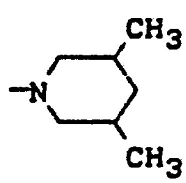 | $n_D^{20}$ 1.5226 |
| 14 | 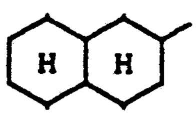 | 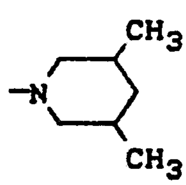 | $n_D^{20}$ 1.5027 |
| 15 | 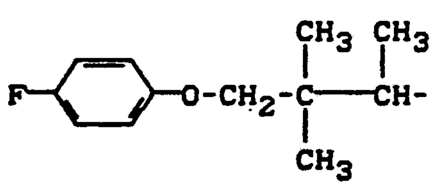 | 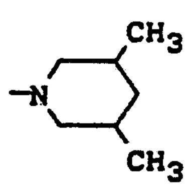 | $n_D^{20}$ 1.5035 |

| Bsp. Nr. | $R^1$ | $-N\langle^{R^2}_{R^3}$ | physikalische Daten |
|---|---|---|---|
| 16 | 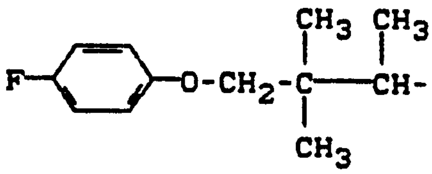 | 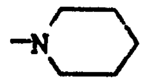 | $n_D^{20}$ 1.5107 |
| 17 | 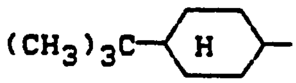 | 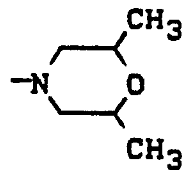 | $n_D^{20}$ 1.4859 |
| 18 | 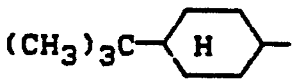 | 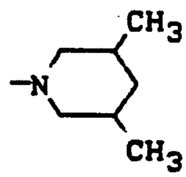 | $n_D^{20}$ 1.4884 |
| 19 | 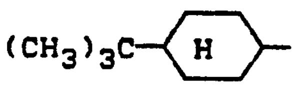 | 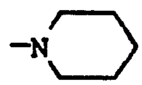 | $n_D^{20}$ 1.4880 |
| 20 | 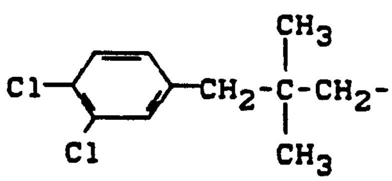 | 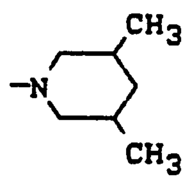 | $n_D^{20}$ 1.5268 |
| 21 | 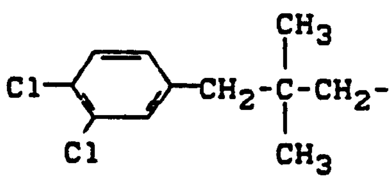 | 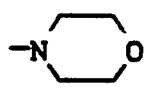 | $n_D^{20}$ 1.5380 |
| 22 | 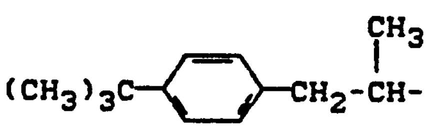 | 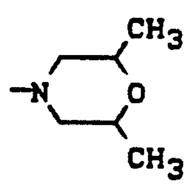 | $n_D^{20}$ 1.5118 |

| Bsp. Nr. | $R^1$ | $-N\langle^{R^2}_{R^3}$ | physikalische Daten |
|---|---|---|---|
| 23 | 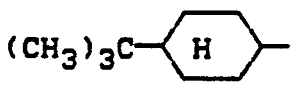 | 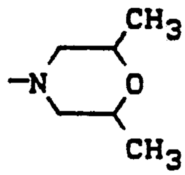 | Fp. 59° C-60° C |
| 24 | 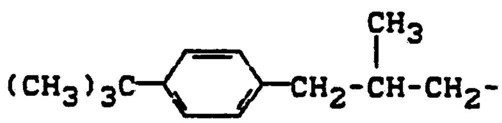 | 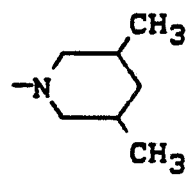 | $n_D^{20}$ 1,5108 |
| 25 | 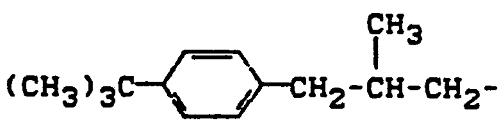 | 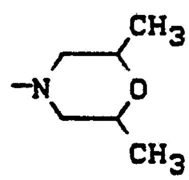 | $n_D^{20}$ 1,5089 |
| 26 | 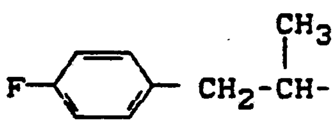 | 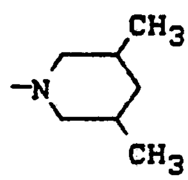 x 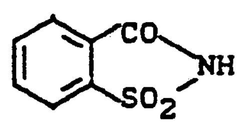 | $^1$H-NMR*): 4,6-4,9 |
| 27 | 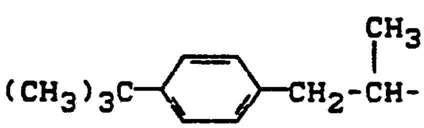 | 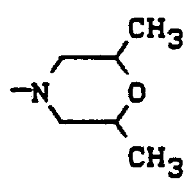 x 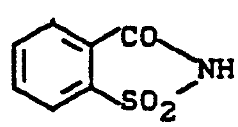 | $^1$H-NMR*): 4,2 |

| Bsp. Nr. | $R^1$ | $-N\langle^{R^2}_{R^3}$ | physikalische Daten |
|---|---|---|---|
| 28 | 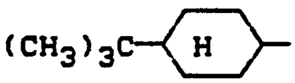 | 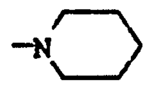 | $^1H$-NMR*): 4,7-4,9 |
| | | x 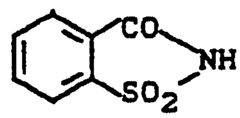 | |
| 29 | 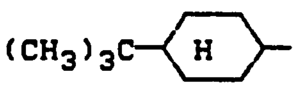 | 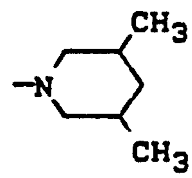 | $^1H$-NMR*): 4,7-4,8 |
| | | x 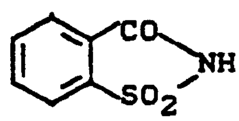 | |
| 30 | 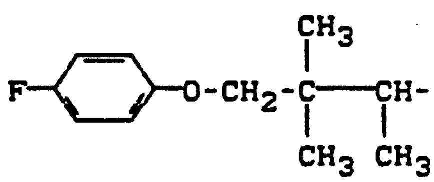 | 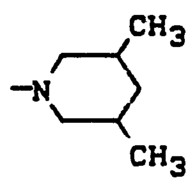 | $^1H$-NMR*): 4,6 |
| | | x 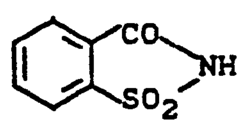 | |
| 31 | 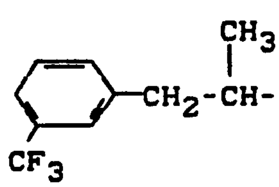 | 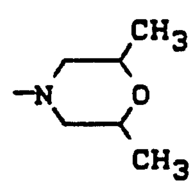 | $^1H$-NMR*): 4,6-4,9 |
| | | x 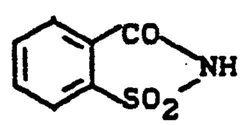 | |

| Bsp. Nr. | $R^1$ | $-N\langle^{R^2}_{R^3}$ | physikalische Daten |
|---|---|---|---|
| 32 | 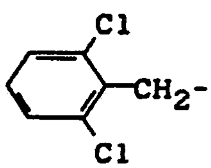 | 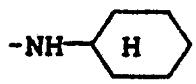 | $n_D^{20}$ 1.5550 |
| 33 | 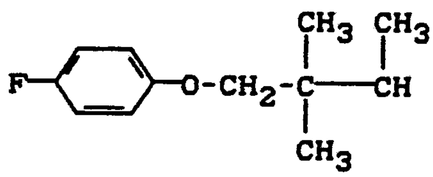 | 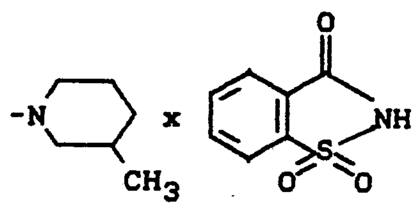 x | 1H-NMR: 0.9-1.1 6.8-7.0 7.55-7.8 |

*) Die $^1$H-NMR-Spektren wurden in $CDCl_3$ mit Tetramethylsilan (TMS) als innerem Standard aufgenommen. Angegeben ist die chemische Verschiebung als δ-Wert in ppm.

Anwendungsbeispiele

In den folgenden Anwendungsbeispielen wurden die nachstehend aufgeführten Verbindungen als Vergleichsubstanzen eingesetzt:

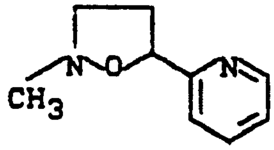

(A)

2-Methyl-5-(2-pyridyl)-isoxazolidin

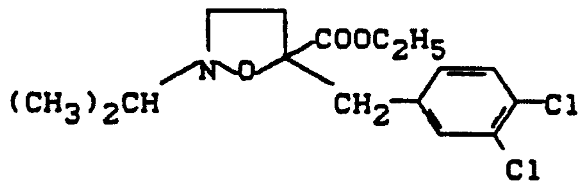

(B)

5-(3,4-Dichlorbenzyl)-5-ethoxycarbonyl-2-isopropyl-isoxazolidin

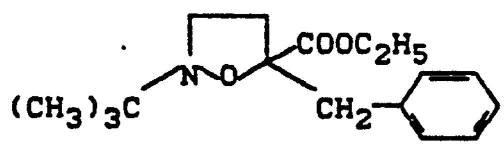

(C)

5-Benzyl-5-ethoxycarbonyl-2-t-butyl-isoxazolidin

(alle bekannt aus DE-OS 34 18 395)

Beispiel A

Erysiphe-Test (Gerste) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp.hordei bestäubt.

Die Pflanzen werden in einem Gewächshaus bei einer Temperatur von ca. 20 °C und einer relativen Luftfeuchtigkeit von ca. 80 % aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigen bei diesem Test z.B. die Verbindungen gemäß den Herstellungbeispielen:

1, 2, 3, 5, 6, 7, 8, 10, 11, 12, 13, 14, 15, 16, 17, 18, 19, 20, 21, 22, 23.

Beispiel B

Pyricularia-Test (Reis) /protektiv

Lösungsmittel: 12,5 Gewichtsteile Aceton

Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25 °C aufgestellt.

4 Tage nach der Inokulation erfolgt die Auswertung des Krankheitsbefalls.

Eine deutliche Überlegenheit in der Wirksamkeit gegenüber dem Stand der Technik zeigen in diesem Test z.B. die Verbindungen gemäß den Herstellungsbeispielen:

3, 8, 10, 16, 21, 22, 23.

**Ansprüche**

1. Aminomethylisoxazolidine der allgemeinen Formel (I),

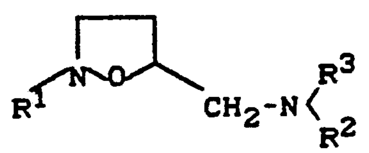

(I)

in welcher

$R^1$ für gegebenenfalls substituiertes Alkyl, für gegebenenfalls substituiertes Cycloalkyl, für gegebenenfalls substituiertes Tetrahydronaphthyl oder für gegebenenfalls substituiertes Decahydronaphthyl steht und

$R^2$ und $R^3$ unabhängig voneinander jeweils für Wasserstoff, für jeweils gegebenenfalls substituiertes Alkyl, Alkenyl, Cycloalkyl oder Aryl stehen oder gemeinsam mit dem Stickstoffatom, an welches sie gebunden sind, für einen gegebenenfalls substituierten, gesättigten Heterocyclus stehen, der gege benenfalls weitere Heteroatome enthalten kann, deren Säureadditionssalze, geometrische und optische Isomere sowie Isomerengemische.

2. Aminomethylisoxazolidine gemäß Anspruch 1, wobei in der Formel (I)

$R^1$ für jeweils geradkettiges oder verzweigtes Alkyl, Hydroxyalkyl, Fluoralkyl, Alkoxyalkyl oder Fluoralkoxyalkyl mit jeweils 1 bis 18 Kohlenstoffatomen und gegebenenfalls 1 bis 9 Fluoratomen steht, außerdem für gegebenenfalls einfach bis mehrfach, gleich oder verschieden im Arylteil substituiertes Arylalkyl, Aryloxalkyl oder Arylthioalkyl mit jeweils 6 bis 10 Kohlenstoffatomen im Arylteil und 1 bis 10 Kohlenstoffatomen im geradkettigen oder verzweigten Alkylteil steht, wobei als Arylsubstituenten jeweils infrage kommen: Halogen, Cyano, Nitro, jeweils geradkettiges oder verzweigtes Alkyl, Alkoxy, Alkylthio, Halogenalkyl, Halogenalkoxy und Halogenalkylthio mit jeweils 1 bis 6 Kohlenstoffatomen und gegebenenfalls 1 bis 9 gleichen oder verschiedenen Halogenatomen, Amino, jeweils geradkettiges oder verzweigtes Alkylamino, Dialkylamino oder Alkoximinoalkyl mit jeweils 1 bis 4 Kohlenstoffatomen in den einzelnen Alkylteilen; außerdem für jeweils gegebenenfalls im Cycloalkylteil einfach bis mehrfach, gleich oder verschieden substituiertes Cycloalkylalkyl oder Cycloalkyl mit jeweils 3 bis 10 Kohlen stoffatomen im Cycloalkylteil und gegebenenfalls 1 bis 6 Kohlenstoffatomen im geradkettigen oder verzweigten Alkylteil steht, wobei als Substituenten jeweils infrage kommen: Hydroxy, jeweils geradkettiges oder verzweigtes Alkyl, Alkoxy, Halogenalkyl oder Halogenalkoxy mit jeweils 1 bis 6 Kohlenstoffatomen und gegebenenfalls 1 bis 9 gleichen oder verschiedenen

Halogenatomen;
für jeweils gegebenenfalls einfach bis mehrfach, gleich oder verschieden substituiertes Tetrahydronaphthyl oder Decahydronaphthyl steht, wobei als Substituenten jeweils infrage kommen: Halogen, jeweils geradkettiges oder verzweigtes Alkyl, Alkoxy, Alkylthio, Halogenalkyl, Halogenalkoxy, Halogenalkylthio und Alkoximinoalkyl mit jeweils 1 bis 4 Kohlenstoffatomen und gegebenenfalls 1 bis 9 gleichen oder verschiedenen Halogenatomen und

$R^2$ und $R^3$ unabhängig voneinander jeweils für Wasser stoff, für jeweils geradkettiges oder verzweigtes Alkyl mit 1 bis 8 Kohlenstoffatomen, Hydroxyalkyl mit 2 bis 8 Kohlenstoffatomen oder Alkoxyalkyl mit 3 bis 8 Kohlenstoffatomen, für geradkettiges oder verzweigtes Alkenyl mit 3 bis 8 Kohlenstoffatomen, für jeweils gegebenenfalls einfach bis mehrfach, gleich oder verschieden durch Alkyl mit 1 bis 4 Kohlenstoffatomen und/oder Halogen substituiertes Cycloalkyl mit 3 bis 7 Kohlenstoff atomen oder für jeweils gegebenenfalls im Arylteil einfach oder mehrfach, gleich oder verschieden substituiertes Aralkyl oder Aryl mit 6 bis 10 Kohlenstoffatomen im Arylteil und gegebenenfalls 1 bis 4 Kohlenstoffatomen im geradkettigen oder verzweigten Alkylteil stehen, wobei als Substituenten jeweils infrage kommen: Halogen, Cyano, Nitro, jeweils geradkettiges oder verzweigtes Alkyl, Alkoxy, Alkylthio, Halogenalkyl, Halogenalkoxy, Halogenalkylthio oder Alkoximinoalkyl mit jeweils 1 bis 4 Kohlenstoffatomen in den einzelnen Alkylteilen und gegebenenfalls 1 bis 9 gleichen oder verschiedenen Halogenatomen oder

$R^2$ und $R^3$ gemeinsam mit dem Stickstoffatom, an welches sie gebunden sind, für einen gegebenenfalls einfach bis mehrfach, gleich oder verschieden substituierten gesättigten 5-bis 7-gliedrigen Heterocyclus stehen, der gegebenenfalls weitere Heteroatome enthalten kann, wobei als Substituenten infrage kommen: jeweils geradkettiges oder verzweigtes Alkyl oder Hydroxyalkyl mit jeweils 1 bis 4 Kohlenstoffatomen, deren Säureadditionssalze, geometrische oder optische Isomere und Isomerengemische.

3. Aminomethylisoxazolidine gemäß Anspruch 1, wobei in der Formel (I)

$R^1$ für geradkettiges oder verzweigtes Alkyl mit 1 bis 12 Kohlenstoffatomen steht; außerdem für im Phenylteil gegebenenfalls ein-bis dreifach, gleich oder verschieden substituiertes Phenylalkyl, Phenoxyalkyl oder Phenylthioalkyl mit jeweils 1 bis 8 Kohlenstoffatomen in den jeweiligen geradkettigen oder verzweigten Alkylteilen steht, wobei als Phenylsubstituenten jeweils infrage kommen:
Fluor, Chlor, Brom, geradkettiges oder verzweigtes Alkyl mit 1 bis 6 Kohlenstoffatomen, Methoxy, Ethoxy, n-oder i-Propoxy, n-, i-, s-oder t-Butoxy, Methylthio, Trifluormethyl, Trifluormethoxy, Trifluormethylthio oder Methoximinomethyl; außerdem für jeweils gegebenenfalls im Cycloalkylteil ein-bis dreifach, gleich oder verschieden substituiertes Cycloalkylalkyl oder Cycloalkyl mit jeweils 5, 6 oder 10 Kohlenstoffatomen im Cycloalkylteil und gegebenenfalls 1 bis 4 Kohlenstoffatomen im geradkettigen oder verzweigten Alkylteil steht, wobei als Substituenten jeweils infrage kommen: geradkettiges oder verzweigtes Alkyl mit 1 bis 5 Kohlenstoffatomen; darüberhinaus für gegebenenfalls ein-bis dreifach, gleich oder verschieden substituiertes Decahydronaphthyl steht, wobei als Substituenten genannt seien: geradkettiges oder verzweigtes Alkyl mit 1 bis 5 Kohlenstoffatomen; und schließlich für gegebenenfalls ein-bis dreifach, gleich oder verschieden substituiertes Tetrahydronaphthyl steht, wobei als Substituenten infrage kommen: geradkettiges oder verzweigtes Alkyl mit 1 bis 5 Kohlenstoffatomen, Methoxy, Trifluormethyl, Trifluormethoxy oder Chlor,

$R^2$ und $R^3$ unabhängig voneinander jeweils für Wasserstoff, Methyl, Ethyl, n-oder i-Propyl, n-, i-, s-oder t-Butyl, n-oder i-Pentyl, n-oder i-Hexyl, Allyl, n-oder i-Butenyl, n-oder i-Pentenyl, Hydroxypropyl, Methoxyethyl, Ethoxyethyl, Methoxypropyl, Ethoxypropyl, Propoxypropyl, für Cyclopropylmethyl, Dichlorcyclopropylmethyl, für Dichlordimethylcyclopropylmethyl, für Dimethylcyclopropylmethyl oder Cyclohexyl stehen oder

$R^2$ und $R^3$ gemeinsam mit dem Stickstoffatom, an welches sie gebunden sind, für einen gegebenenfalls ein-bis dreifach, gleich oder verschieden substituierten Heterocyclus der Formel

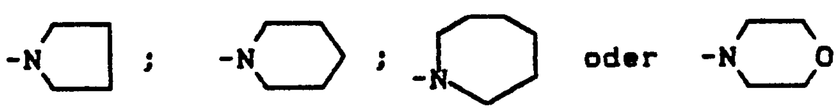

stehen, wobei als Substituenten infrage kommen: Methyl, Ethyl und Hydroxymethyl, und deren Säureadditionssalze, geometrische oder optische Isomere und Isomerengemische.

4. Verfahren zur Herstellung von Aminomethylisoxazolidinen der allgemeinen Formel (I),

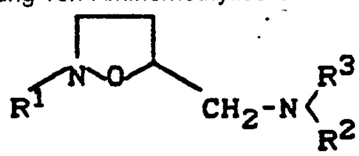 (I)

in welcher
$R^1$ für gegebenenfalls substituiertes Alkyl, für gegebenenfalls substituiertes Cycloalkyl, für gegebenenfalls substituiertes Tetrahydronaphthyl oder für gegebenenfalls substituiertes Decahydronaphthyl steht und
$R^2$ und $R^3$ unabhängig voneinander jeweils für Wasserstoff, für jeweils gegebenenfalls substituiertes Alkyl, Alkenyl, Cycloalkyl oder Aryl stehen oder gemeinsam mit dem Stickstoffatom, an welches sie gebunden sind, für einen gegebenenfalls substituierten, gesättigten Heterocyclus stehen, der gegebenenfalls weitere Heteroatome enthalten kann,
sowie deren Säureadditionssalze, dadurch gekennzeichnet, daß man
(a) Hydroxylamin-Derivate der Formel (II),

$R^1$-NH-OH (II)

in welcher
$R^1$ die oben angegebene Bedeutung hat,
mit Allylaminen der Formel (III),

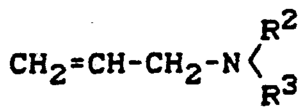 (III)

in welcher
$R^2$ und $R^3$ die oben angegebene Bedeutung haben,
in Gegenwart von Formaldehyd sowie gegebenenfalls in Gegenwart eines Verdünnungsmittels umsetzt, oder
(b) Isoxazolidine der Formel (IV),

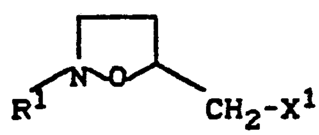 (IV)

in welcher
$R^1$ die oben angegebene Bedeutung hat und
$X^1$ für eine elektronenanziehende Abgangsgruppe steht,
mit Aminen der Formel (V),

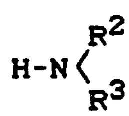 (V)

in welcher
$R^2$ und $R^3$ die oben angegebene Bedeutung haben,
gegebenenfalls in Gegenwart eines Verdünnungsmittels und gegebenenfalls in Gegenwart eines Säurebindemittels sowie gegebenenfalls in Gegenwart eines Katalysators umsetzt oder
(c) die nach Verfahren (a) oder (b) erhältlichen Aminomethylisoxazolidine der Formel (Ia),

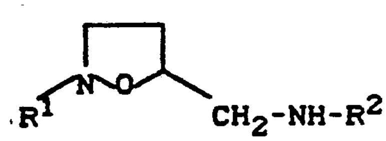 (Ia)

in welcher
$R^1$ und $R^2$ die oben angegebene Bedeutung haben, mit Alkylierungsmitteln der Formel (VI),

$R^{3-1}$-$X^2$ (VI)

in welcher
$R^{3-1}$ für jeweils gegebenenfalls substituiertes Alkyl, Alkenyl oder Cycloalkyl steht und
$X^2$ für eine elektronenanziehende Abgangsgruppe steht,

gegebenenfalls in Gegenwart eines Verdünnungsmittels und gegebenenfalls in Gegenwart eines Säurebindemittels sowie gegebenenfalls in Gegenwart eines Katalysators umsetzt und gegebenenfalls anschließend eine Säure addiert.

5. Schädlingsbekämpfungsmittel, gekennzeichnet durch einen Gehalt an mindestens einem Aminomethylisoxazolidin der Formel (I) gemäß den Ansprüchen 1 und 4.

6. Verwendung von Aminomethylisoxazolidinen der Formel (I) gemäß den Ansprüchen 1 und 4 zur Bekämpfung von Schädlingen.

7. Verwendung von Aminomethylisoxazolidinen der Formel (I) gemäß Anspruch 6 zur Bekämpfung von Pilzen.

8. Verfahren zur Bekämpfung von Schädlingen, dadurch gekennzeichnet, daß man Aminomethylisoxazolidine der Formel (I) gemäß den Ansprüchen 1 und 4 auf Schädlinge oder ihren Lebensraum einwirken läßt.

9. Verfahren zur Herstellung von Schädlingsbekämpfungsmitteln, dadurch gekennzeichnet, daß man Aminomethylisoxazolidine der Formel (I) gemäß den Ansprüchen 1 und 4 mit Streckmitteln und/oder oberflächenaktiven Mitteln vermischt.

10. Hydroxylamine der Formel (IIa),

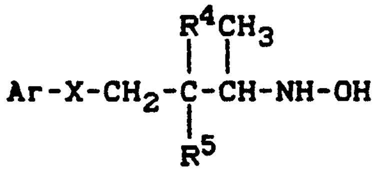

(IIa)

in welcher

Ar für gegebenenfalls substituiertes Phenyl steht, wobei als Substituenten infrage kommen: Halogen, Cyano, Nitro, jeweils geradkettiges oder verzweigtes Alkyl, Alkoxy, Alkylthio, Halogenalkyl, Halogenalkoxy und Halogenalkylthio mit jeweils 1 bis 6 Kohlenstoffatomen und gegebenenfalls 1 bis 9 gleichen oder verschiedenen Halogenatomen, Amino, jeweils geradkettiges oder verzweigtes Alkylamino, Dialkylamino oder Alkoximinoalkyl mit jeweils 1 bis 4 Kohlenstoffatomen in den einzelnen Alkylteilen;

X für Sauerstoff oder Schwefel steht und

$R^4$ und $R^5$ unabhängig voneinander jeweils für Alkyl mit 1 bis 3 Kohlenstoffatomen stehen.